# EUROPEAN PATENT SPECIFICATION

(11) **EP 2 068 960 B1**
(45) Date of publication and mention of the grant of the patent: **15.02.2012**
(21) Application number: 07811549.0
(22) Date of filing: 24.08.2007
(51) Int. Cl.: A61L 29/06, A61L 29/18, A61L 31/06

(54) **POLYCARBONATE POLYURETHANE VENOUS ACCESS DEVICES**
VENENZUGANGSVORRICHTUNGEN AUS POLYCARBONAT-POLYURETHAN
DISPOSITIF D'ACCÈS VEINEUX EN POLYCARBONATE POLYURÉTHANE

(30) Priority: 24.08.2006 US 839949 P; 23.08.2007 US 895192
(43) Date of publication of application: 17.06.2009
(60) Divisional application: 12150813.9
(73) Proprietor: Navilyst Medical, Inc., Marlborough, MA 01752 (US)
(72) Inventor: MURPHY, Nathan, New South Wales, 2777 (AU); GIRARD, Mark, Medway, MA 02053 (US); FISK, Thomas, Natick, MA 01760 (US)
(74) Representative: Pfenning, Meinig & Partner GbR
(86) International application number: PCT/US2007/018848
(87) International publication number: WO 2008/024514

(56) References cited:
- WO-A-2004/058324
- WO-A-2006/058042
- US-A- 5 112 312
- US-A1- 2004 171 747

## Description

### BACKGROUND OF THE INVENTION

In current medical practice, it is commonly necessary to introduce a catheter into the central venous circulation (CVC) system for various purposes. For example, catheters may be introduced for purposes of delivering fluids, such as blood, glucose solutions, medications, diagnostic agents, and so forth, to the vasculature. Catheters may also be introduced for purposes of withdrawing blood from the vasculature, for example, in order to treat the blood, to carry out diagnostics on the blood, and so forth.
US2004/171747 discloses a medical device such as a catheter comprising a coating made of a polycarbonate polyurethane aqueous emulsion.
WO2006/058042 discloses a device such as a catheter comprising a shaft made of a polycarbonate polyurethane copolymer matrix.

### SUMMARY OF THE INVENTION

A central venous access device comprising an extruded catheter shaft having one or more lumens, said catheter shaft consisting of a polycarbonate polyurethane having a molecular weight in excess of 90,000 g/mol.

### BRIEF DESCRIPTION OF THE DRAWINGS

Fig 1A is a longitudinal view of a PICC catheter, which is formed from a polycarbonate polyurethane in accordance with the present invention.

Fig. 1B is a view of the proximal end of the catheter shaft of Fig. 1A.

Fig. 1C is a view of the distal end of the catheter shaft of Fig. 1A.

Fig. 2 is a perspective view of the catheter shaft of Figs. 1A-1C in conjunction with an assembly, which includes a hub, an extension tube, and a pressure activated safety valve (PASV).

Fig. 3 illustrates the effects of solvent vapor and of polycarbonate polyurethane molecular weight upon cycles to failure.

Fig. 4 illustrates the effects of environmental conditions and of polycarbonate polyurethane molecular weight upon cycles to failure.

Fig. 5 illustrates variation in cycles to failure among various samples after three hours exposure to alcohol.

### DETAILED DESCRIPTION

As used herein, a "catheter" is a medical device that includes a flexible shaft; which contains one (including annular shafts, i.e., tubes) or more lumens, and which may be inserted into the body for introduction of fluids, for removal of fluids, or both.

A catheter may further include various accessory components, for example, molded components, over-molded sub-assemblies, connecting fittings such as hubs, extension tubes, and so forth. Various catheter tips designs are known, including stepped tips, tapered tips, over-molded tips and split tips (for multilumen catheters), among others.

A "central venous access catheter" is a catheter that provides access to the central venous circulation system.

Central venous access may be achieved by direct puncture of the central venous circulation system, e.g., via the internal jugular vein, subclavian vein or femoral vein. Catheters of this type, known as "central catheters" or "central venous catheters," are relatively short, and can generally remain in place for only a short time (e.g., generally less than 7 days).

Other catheters have also been developed which can be inserted into peripheral veins (e.g., the antecubital, basilica, or cephalic vein) and advanced to access the central venous system, with the tip commonly positioned in the superior vena cava or right atrium, thus allowing for rapid dilution of infused fluids. These devices avoid difficulties associated with the direct puncture of the central venous circulation system, and they allow for long term (e.g., 180 days or more) and repeated access to a patient's vascular system, thereby avoiding multiple injections and minimizing trauma and pain to the patient.

Specific examples of catheters of this type include so-called peripherally inserted central catheters ("PICCs"), midline catheters, and peripheral catheters. A typical PICC, midline, or peripheral catheter contains a thin, flexible shaft, which contains one or more lumens and which terminates at the proximal end with a suitable fitting, such as a hub or other fitting. The primary difference between these three devices is the length of the tubing, with the peripheral catheter being the shortest and the PICC being the longest. The rationale for different lengths is driven by the type and duration of the therapy a patient is to receive.

Hemodialysis catheters are another important class of central venous access catheters. Hemodialysis catheters are commonly multi-lumen catheters in which one lumen is used to carry blood from the body to a dialysis machine, and another lumen returns blood to the body. Central venous access may be attained by puncture of various major blood vessels, including the internal jugular vein, subclavian vein, or femoral vein.

Central venous access may also be provided via venous access ports. These specialized catheters typically have the three following components: (a) a catheter, (b) a reservoir, typically formed of a metal or polymer, which holds a small amount of liquid and which is connected to the catheter, and (c) a septum, which covers the reservoir and allows access to the reservoir upon insertion of a needle. The reservoir and covering septum are surgically placed under the skin of the chest or arm, and the catheter extends into a central vein.

Catheter shafts for central venous catheters such as those describe above, among others, are typically made from polymers. Suitable polymers are those that can be formed into tubing that is flexible enough to be routed through the vasculature without causing trauma to the patient. When formed into tubing, the polymer chosen should also provide strength sufficient to ensure that the lumen does not collapse in the vasculature, and should resist repeated flexure. Polyurethane-based polymers are commonly employed to meet these criteria.

In general, polyurethanes are a family of polymers that are synthesized from polyfunctional isocyanates (e.g., diisocyanates, including both aliphatic and aromatic diisocyanates) and polyols (also, referred to as macroglycols, e.g., macrodiols). Commonly employed macroglycols include polyester glycols, polyether glycols and polycarbonate glycols. Typically, aliphatic or aromatic diols are also employed as chain extenders, for example, to impart the useful physical properties described above. Examples of diol chain extenders include butane diol, pentane diol, hexane diol, heptane diol, benzene dimethanol, hydraquinone diethanol and ethylene glycol.

Polyurethanes are commonly classified based on the type of macro glycol employed, with those containing polyester glycols being referred to as polyester polyurethanes, those containing polyether glycols being referred to as polyether polyurethanes, and those containing polycarbonate glycols being referred to as polycarbonate polyurethanes. Polyurethanes are also commonly designated aromatic or aliphatic on the basis of the chemical nature of the diisocyanate component in their formulation.

Preferred polyurethanes for the practice of the present invention are polycarbonate polyurethanes. For example, U.S. Patent Appln. No. 2004/0131863 to Belliveau et al., describes aliphatic polycarbonate polyurethanes which are the reactions products of (a) a hydroxyl terminated polycarbonate, (b) an aliphatic diisocyanate and (c) a lower aliphatic chain extender. Hydroxyl terminated polycarbonate polyol may be prepared by reacting a glycol with a carbonate, as disclosed in U.S. Pat. No. 4,131,731. Suitable aliphatic diisocyanates include hexamethylene diisocyanate (HDI), isophorone diisocyanate (IPDI), trimethyl hexamethylene diisocyanate (TMHDI), dicyclohexyl methane diisocyanate (HMDI), and dimer acid diisocyanate (DDI), with HMDI said to be preferred. Suitable chain extenders include lower aliphatic glycols having from about 2 to about 10 carbon atoms, such as, for instance ethylene glycol, diethylene glycol, propylene glycol, dipropylene glycol, 1,4-butanediol, 1,6-hexanediol, 1,3-butanediol, 1,5-pentanediol, 1,4-cyclohexanedimethanol hydroquinone di(hydroxyethyl) ether, neopentyglycol, and the like, with 1,4-butanediol said to be preferred.

Polycarbonate polyurethanes are strong, allowing catheters to be formed with thinner walls, regardless of whether the catheter shaft is a single lumen shaft or a multi-lumen shaft. Subsequently, catheters made from these materials may be formed with smaller ODs as compared, for example to other catheter materials such as silicone, or they may be formed having the same OD, but with a larger ID, and therefore having a greater flow rate. Wall thickness for polycarbonate polyurethane catheter shafts will vary with application and may range, for example, from 0.003" to 0.005" to 0.01" to 0.015", among other thicknesses.

Moreover, while being sufficiently flexible to avoid trauma to the patient, these materials are stiff as compared, for example, to silicone, which helps with insertion of the catheter.

These materials are also thermoplastics, meaning that a variety of thermoplastic processing techniques, such as extrusion, molding, and so forth, may be employed to form medical devices and medical device components, including catheter shafts, from the same.

Kink resistance (i.e., resistance to failure as a result of repeated kinking), is also an important property of catheter shaft materials. In general, the kink resistance of polycarbonate polyurethanes lessens as the walls of the catheter shaft become thinner and thinner. Moreover, kink resistance also lessens significantly upon exposure to certain materials such as alcohol (i.e., ethanol, isopropyl alcohol, etc.) and alcohol-containing materials such as Chloroprep® (a product commonly used for skin preparation, which contains 2% chlorhexadine gluconate and 70% isopropyl alcohol), among other materials. Hence, kink resistance is particularly important for central venous access devices such as PICCs, which have thin catheter walls and which are subjected to repeated exposure to alcohol at the entrance site of the body.

The present inventors have unexpectedly found that by employing an extruded catheter shaft which comprises a polycarbonate polyurethane having a weight average molecular weight in excess of 90,000 g/mol, that superior kink resistance is obtained, even upon exposure to substantial amounts of alcohol. Weight average molecular weight may be within the range, for example, of 90,000 to 95,000 to 100,000 to 105,000 to 110,000 or more.

To be visible under x-ray (e.g., by x-ray fluoroscopy), the catheter shaft may be rendered more absorptive of x-rays than the surrounding tissue. Examples of radiopaque agents include metals, metal salts, metal oxides, and iodinated compounds. More specific examples of such contrast agents include gold, tungsten, platinum, tantalum, iridium, or other dense metal, barium sulfate, bismuth subcarbonate, bismuth trioxide, bismuth oxychloride, metrizamide, iopamidol, iothalamate sodium, iodomide sodium, and meglumine. For example, in certain embodiments, the catheter shaft will further contain from 30 to 50 wt% barium, more preferably about 40 wt% barium.

In certain embodiments, the catheter shaft catheter shaft has a durometer value ranging from 50A to 100A, more preferably 75A to 85A, among other values.

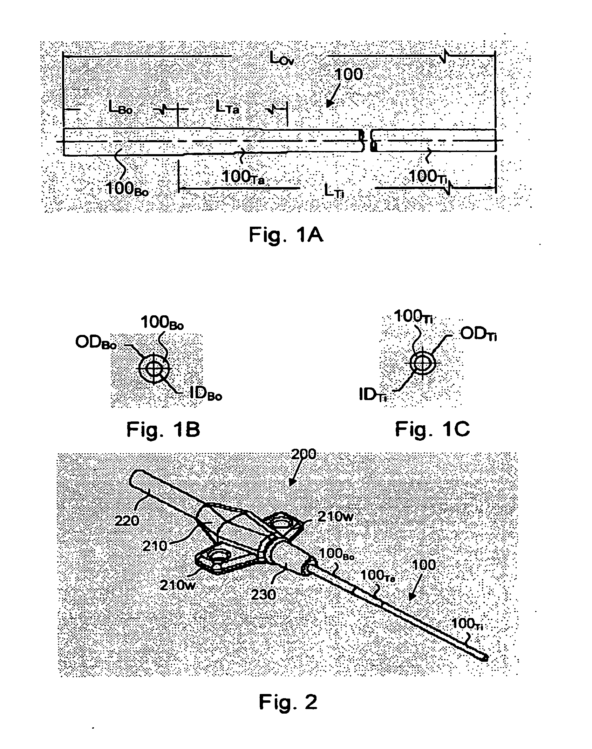

A PICC catheter shaft 100 (also referred to herein as an "extrusion"), formed from a polycarbonate polyurethane polycarbonate polyurethane having a molecular weight in excess of 90,000, is illustrated in Fig. 1A. Fig 1B is a view of the proximal (left-hand) end of the shaft 100 of Fig. 1A. Fig 1C is a view of the distal (right-hand) end of the shaft 100.

The shaft 100 includes a body section 100_{Bo}, having length L_{Bo}. The body section 100_{Bo} also has an outer diameter OD_{Bo} and an inner diameter (lumen diameter) ID_{Bo} at its proximal end. The shaft 100 further includes a tip section 100_{Ti}. At its distal end, the tip section 100_{Ti} has an outer diameter OD_{Ti} and an inner diameter (lumen diameter) ID_{Ti}. The shaft 100 further has a tapered section 100_{Ta}, having length L_{Ta}. L_{Ti} is the combined length of the tip section 100_{Ti} and the tapered section 100_{Ta}. The overall length is L_{Ov}. Typical dimensions for some of these values are provided in Table I below:

**Table 1.**

| **Catheter Type** | 4-3F | 6-4F | 6-5F |
|---|---|---|---|
| **L_{Bo} (cm)** | 7.5 min. | 7.5 min. | 7.5 min. |
| **OD_{Bo} (in)** | .053 ± .003 | .079 ± .004 | 079 ± .004 |
| **ID_{Bo} (in)** | .028 ±.002 | .047 ± .003 | .047 ± .003 |
| **L_{Ti} (cm)** | 65 min. | 65 min. | 65 min. |
| **OD_{Ti} (in)** | .040 ± .003 | .053 ± .003 | .066 ± .003 |
| **ID_{Ti} (in)** | .025 ± .002 | .034 ±.002 | .044 ± .0025 |
| **L_{Ta} (cm)** | 5 max. | 5 max. | 5 max. |
| **Wall Thickness (in)** | .003 min. | 0.003 min. | 0.003 min. |

In Fig. 2, the catheter shaft 100 of Fig. 1, is shown in combination with an assembly 200, which includes hub 210, which has suture wings 210w, extension tube 220, and pressure activated safety valve 230 (PASV).

Although the catheter shaft shown in Figs. 1A-C and 2 is a single lumen shaft, shafts with multiple lumens (e.g., two, three, four, or even more) may be formed as noted above. For example a dual lumen catheter shaft may be formed and placed in an assembly along with dual extension tubes as well as an appropriate hub and a valve, if desired.

### EXAMPLES

Catheter shafts (extrusions) are formed using a range of Carbothane® resins having molecular weights of 76,000, 87,000, 102,000 and 110,000 g/mol from Noveon, Inc., Cleveland, Ohio.

Extrusions of OD's ranging from 4Fr. (French) to 7Fr. were cut to approximately 2.75 inches in length for testing. Where the extrusion is tested in conjunction with an assembly, such as that described in Fig. 2, the extrusion is cut 4.5 cm from the suture wing and the extension tube is cut 1 cm from the suture wing.

Samples are exposed to vapor in a sealed glass jar by placing them on aluminum blocks above a solution of 70% alcohol or Chloroprep® for a designated time. Temperature is controlled by immersing the jar halfway in a water bath at 37 ± 2°C.

Axial Cycling Tester (built in house) is used for kink resistance testing. The distance between the grippers is set in the up and down positions such that the sample kinks when in the down position and undergoes 25% elongation.

Where an extrusion is tested in conjunction with an assembly, ten kinks are formed at the suture wing prior to testing. Also a three minute kink at body temperature is performed prior to testing.

The speed controller is adjusted as necessary to ensure that it is operating at 200 ± 5 cycles per minute. The machine is stopped every 1000 cycles and the sample is examined at both kink locations for cracks or holes or for any other sign of failure.

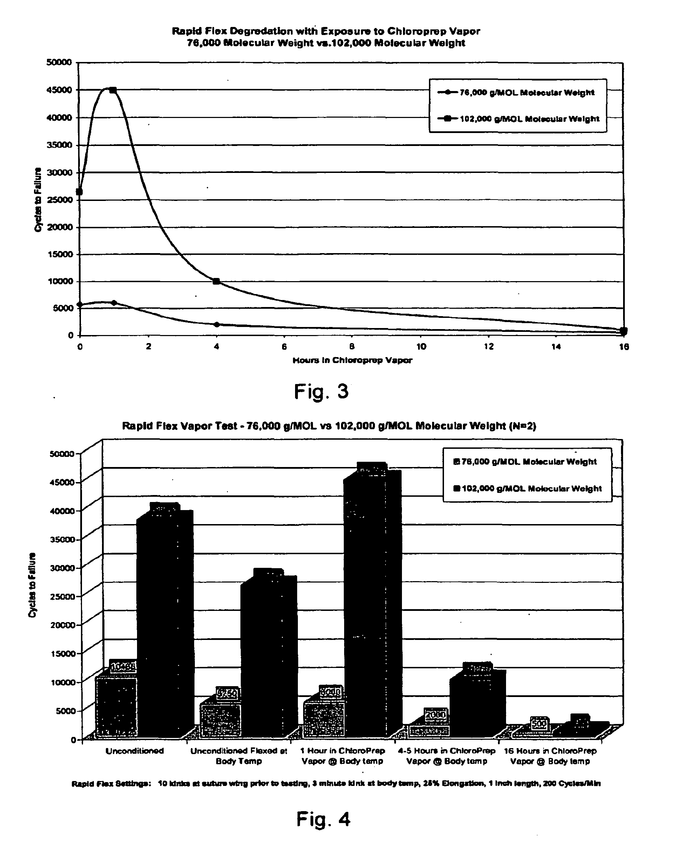

Fig. 3 illustrates the effect of solvent vapor upon cycles to failure for extrusion and extrusion to suture wing interface. As can be seen, a small amount of vapor exposure (1 hr) actually increases the resistance of the sample to failure. However, this benefit is quickly lost. As also seen from this figure, the 102,000 mol. wt. sample outperformed the 76,000 mol. wt. sample in all cases.

These data are also displayed in a bar-graft format in Fig. 4. Also shown is cycles to failure data where the samples are flexed at body temperature.

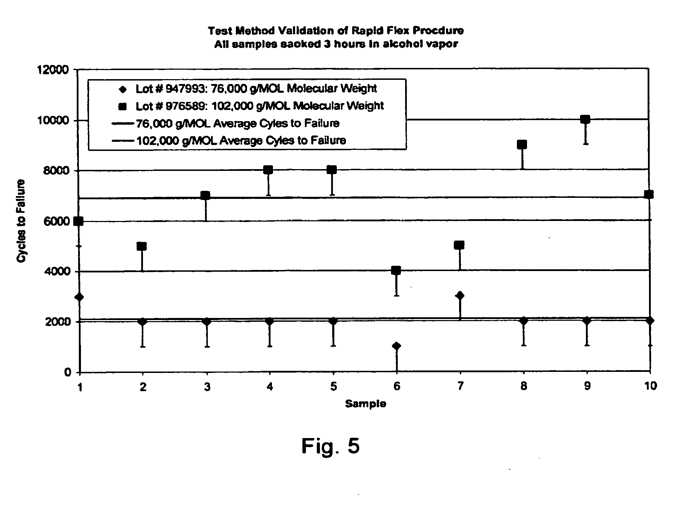

Fig. 5 shows sample variation among ten 102,000 mol. wt. extrusions and ten 76,000 mol. wt. extrusions after three hours exposure to alcohol (70% isopropanol). Average cycles to failure is more than three times as high for the 102,000 mol. wt. samples as compared to the 76,000 mol. wt. samples.

Table 2 below shows maximum, minimum, and average cycles to failure, both before and after sterilization two times in ethylene oxide using standard protocol for the following: twenty-one 102,000 mol. wt. extrusions, twenty-two 110,000 mol. wt. extrusions, and twenty-two 87,000 mol. wt. extrusions. This table again illustrates the advantages of increasing molecular weight vis-à-vis kink resistance.

**Table 2.**

| Sample Identification | Average Cycles to Failure | Maximum Cycles to Failure | Minimum Cycles to Failure |
|---|---|---|---|
| Pre-Sterile 87kMW | 6271 cycles | 8001 cycles | 4670 cycles |
| Post 2x-Sterile 87kMW | 4801 cycles | 6004 cycles | 3002 cycles |
| Pre-Sterile 102kMW | 15,275 cycles | 23,002 cycles | 6000 cycles |
| Post 2x-sterile 102kMW | 17,620 cycles | 19,000 cycles | 7000 cycles |
| Pre-Sterile 110kMW | 15,184 cycles | 24,004 cycles | 8003 cycles |
| Post 2x-sterile 110kMW | 17,456 cycles | 26,005 cycles | 7000 cycles |

## Claims

1. A central venous access device comprising an extruded catheter shaft having one or more lumens, said catheter shaft consisting of a polycarbonate polyurethane having a weight average molecular weight in excess of 90,000 g/mol.

2. The central venous access device of claim 1, wherein said polycarbonate polyurethane is an aliphatic polycarbonate polyurethane.

3. The central venous access device of claim 1, wherein said molecular weight is in excess of 95,000 g/mol.

4. The central venous access device of claim 1, wherein said molecular weight is in excess of 100,000 g/mol.

5. The central venous access device of claim 1, wherein the catheter shaft comprises a single lumen.

6. The central venous access device of claim 5, wherein said catheter shaft is in the form of an annulus.

7. The central venous access device of claim 1, wherein said catheter shaft comprises multiple lumens.

8. The central venous access device of claim 1, wherein said device is a central catheter.

9. The central venous access device of claim 1, wherein said device is a peripheral catheter.

10. The central venous access device of claim 1, wherein said device is a midline catheter.

11. The central venous access device of claim 1, wherein said device is a hemodialysis catheter.

12. The central venous access device of claim 1, wherein said device is a peripherally inserted central catheter.

13. The central venous access device of claim 12, wherein said catheter shaft has a minimum wall thickness of ranging from 0,381 mm (0.015 inch) to 0,0762 mm (0.003 inch).

14. The central venous access device of claim 1, wherein said device is, a venous access port.

15. The central venous access device of claim 1, wherein said catheter shaft
a) is an extruded catheter shaft or
b) is a molded catheter shaft or
c) has a durometer value ranging from 75 shore A to 85 shore A.

## Patentansprüche

1. Zentralvenen-Zugangsvorrichtung, aufweisend eine extrudierte Katheterwelle mit einem oder mehreren Lumen, welche Katheterwelle aus einem Polycarbonat-Polyurethan mit einem durchschnittlichen Molekulargewicht oberhalb 90000 g/mol besteht.

2. Zentralvenen-Zugangsvorrichtung nach Anspruch 1, bei der das Polycarbonat-Polyurethan ein aliphatisches Polycarbonat-Polyurethan ist.

3. Zentralvenen-Zugangsvorrichtung nach Anspruch 1, bei der das Molekulargewicht oberhalb 95000 g/mol ist.

4. Zentralvenen-Zugangsvorrichtung nach Anspruch 1, bei der das Molekulargewicht oberhalb 100000 g/mol ist.

5. Zentralvenen-Zugangsvorrichtung nach Anspruch 1, bei der die Katheterwelle ein einzelnes Lumen aufweist.

6. Zentralvenen-Zugangsvorrichtung nach Anspruch 5, bei der die Katheterwelle in der Form eines Kreisrings ist.

7. Zentralvenen-Zugangsvorrichtung nach Anspruch 1, bei der die Katheterwelle mehrere Lumen aufweist.

8. Zentralvenen-Zugangsvorrichtung nach Anspruch 1, bei der die Vorrichtung ein Zentralkatheter ist.

9. Zentralvenen-Zugangsvorrichtung nach Anspruch 1, bei der die Vorrichtung ein peripherer Katheter ist.

10. Zentralvenen-Zugangsvorrichtung nach Anspruch 1, bei der die Vorrichtung ein Mittellinien-Katheter ist.

11. Zentralvenen-Zugangsvorrichtung nach Anspruch 1, bei der die Vorrichtung ein Hämodialyse-Katheter ist.

12. Zentralvenen-Zugangsvorrichtung nach Anspruch 1, bei der die Vorrichtung ein peripher eingeführter Zentralkatheter ist.

13. Zentralvenen-Zugangsvorrichtung nach Anspruch 12, bei der die Katheterwelle eine minimale Wanddicke im Bereich von 0,381 mm (0,015 Zoll) bis 0,0762 mm (0,003 Zoll) hat.

14. Zentralvenen-Zugangsvorrichtung nach Anspruch 1, bei der die Vorrichtung ein Venenzugangstor ist.

15. Zentralvenen-Zugangsvorrichtung nach Anspruch 1, bei der die Katheterwelle
a) eine extrudierte Katheterwelle ist, oder
b) eine geformte Katheterwelle ist, oder
c) einen Härtewert im Bereich von 75 Shore A bis 85 Shore A hat.

## Revendications

1. Dispositif d'accès veineux central comprenant une tige de cathéter extrudée présentant une ou plusieurs lumières, ladite tige de cathéter étant constituée d'un polycarbonate polyuréthane ayant un poids moléculaire moyen en poids excédant 90 000 g/mol.

2. Dispositif d'accès veineux central selon la revendication 1, dans lequel ledit polycarbonate polyuréthane est un polycarbonate polyuréthane aliphatique.

3. Dispositif d'accès veineux central selon la revendication 1, dans lequel ledit poids moléculaire excède 95 000 g/mol.

4. Dispositif d'accès veineux central selon la revendication 1, dans lequel ledit poids moléculaire excède 100 000 g/mol.

5. Dispositif d'accès veineux central selon la revendication 1, dans lequel la tige de cathéter comprend une seule lumière.

6. Dispositif d'accès veineux central selon la revendication 5, dans lequel ladite tige de cathéter se présente sous la forme d'un espace annulaire.

7. Dispositif d'accès veineux central selon la revendication 1, dans lequel ladite tige de cathéter comprend plusieurs lumières.

8. Dispositif d'accès veineux central selon la revendication 1, dans lequel ledit dispositif est un cathéter central.

9. Dispositif d'accès veineux central selon la revendication 1, dans lequel ledit dispositif est un cathéter périphérique.

10. Dispositif d'accès veineux central selon la revendication 1, dans lequel ledit dispositif est un cathéter médian.

11. Dispositif d'accès veineux central selon la revendication 1, dans lequel ledit dispositif est un cathéter d'hémodialyse.

12. Dispositif d'accès veineux central selon la revendication 1, dans lequel ledit dispositif est un cathéter central inséré par voie périphérique.

13. Dispositif d'accès veineux central selon la revendication 12, dans lequel ladite tige de cathéter a une épaisseur minimale de paroi variant de 0,381 mm (0,015 pouce) à 0,0762 mm (0,003 pouce).

14. Dispositif d'accès veineux central selon la revendication 1, dans lequel ledit dispositif est un orifice d'accès veineux.

15. Dispositif d'accès veineux central selon la revendication 1, dans lequel ladite tige de cathéter
a) est une tige de cathéter extrudée ou
b) est une tige de cathéter moulée ou
c) a une durée variant de 75 Shore A à 85 Shore A.
